Europäisches Patentamt

European Patent Office

Office européen des brevets

(11) Publication number: **0 089 932**
**A1**

# (12) EUROPEAN PATENT APPLICATION

(21) Application number: 83830060.6

(22) Date of filing: 18.03.83

(51) Int. Cl.³: **A 61 B 6/00**
**B 60 P 3/00**

(30) Priority: 22.03.82 IT 2031182

(43) Date of publication of application:
28.09.83 Bulletin 83/39

(84) Designated Contracting States:
AT BE CH DE FR GB LI LU NL SE

(71) Applicant: **Poy, Carlo**
**Via S. Saint Bon, 7**
**I-20147 Milan(IT)**

(72) Inventor: **Poy, Carlo**
**Via S. Saint Bon, 7**
**I-20147 Milan(IT)**

(74) Representative: **Notarbartolo, Manfredi et al,**
**Studio Brevetti e Marchi NOTARBARTOLO & GERVASI**
**Viale Bianca Maria, 33**
**I-20122 Milano(IT)**

(54) Medical complex constituted by a radiological unit mounted and assembled in an off-the-road automobile.

(57) This invention relates to a medical radiological complex for field use, hereinafter also known for brevity as "complex", constituted by a medical radiological unit of the most advanced type, hereinafter also known for brevity as "radiological unit", and an off-the-road automobile generally of standard type.

The radiological unit is mounted and assembled in the automobile. The purpose of this complex, which is installed in proximity to war zones or injured persons by the responsible personnel, is to facilitate identification of the injuries and traumas suffered by the wounded, and to arrange for their treatment or despath to nearby hospitals which are most appropriate in terms of location and specialisation.

EP 0 089 932 A1

./...

FIG.2

## DESCRIPTION

This invention relates to a complex comprising a radiological unit and an off-the-road automobile, the former being mounted and assembled in the latter.

The purpose of the present invention is to move the automobile as close as possible to a zone containing traumatised, injured or wounded persons in general, and then select those wounded showing injuries identified by the radiological examination from those requiring other treatment and operations.

In this manner, it is possible either to treat the wounded on the spot or to despatch them to hospitals specialised in the treatment of their particular injuries, traumas etc.

At the present time, the wounded are generally attended to by loading them on to coaches fitted with conventional radiological apparatus.

The use of such coaches is obviously limited to roads suitable for motor traffic, and this is a serious defect. In addition, such coaches are heavy, bulky, and can perform their function substantially only in favourable environmental conditions.

This invention relates to a radiological unit of advanced type mounted and assembled in an off-the-road automobile.

As such automobiles are well known, the description will be limited to the radiological unit used herein.

Said unit is generally constituted by an X-ray generating source, an image intensifier, a camera, an image vision monitor, a supplementary TV monitor, controls for the X-ray unit, a mobile support with rapid manual control, a bed for transporting the patient, an electrical generator, and aiming handles.

A suitable store of water is also available.

All the described apparatus are suitably connected together and to the automobile, and are made operational, their operation all being within the range of the expert, and not requiring description.

The invention has the following characteristics, coupled with the following advantages over present-day radiological and radiographic ambulances, for the following reasons:

- it is particularly suitable for use in the mass radiological investigation of a large number of unconscious subjects resulting from war or catastrophe;

- it is suitable for making a radiographic examination of the entire body of the patient by successive pulsed flash radioscopy.   It is possible simultaneously to record the video image on tape, so making it independent of the conventional radiographic documentation systems on radiographic film.   Development, fixing and drying apparatus requiring a dark-room can thus be dispensed with;

- said recording can be projected immediately on to the screen, or can be subsequently processed with reproduction either on a photograph or on film;

- the cathode ray tube can be located either under a tent or in the open air, and can be positioned variously relative to the patient.   It thus becomes possible to carry out an extremely easy examination which has a minimum traumatising effect, even on high risk patients suffering from shock.

The invention therefore differs completely from the radiological ambulances and radiographic units used at the present time, and has further advantages over the known types, namely a limited overall size, reduced vulnerability, and the ability to reach

localities inaccessible to other means, either directly or carried by a helicopter.

The radiological complex mounted and assembled in an off-the-road automobile according to the present invention, is illustrated by non-limiting example in the accompanying figures in which:

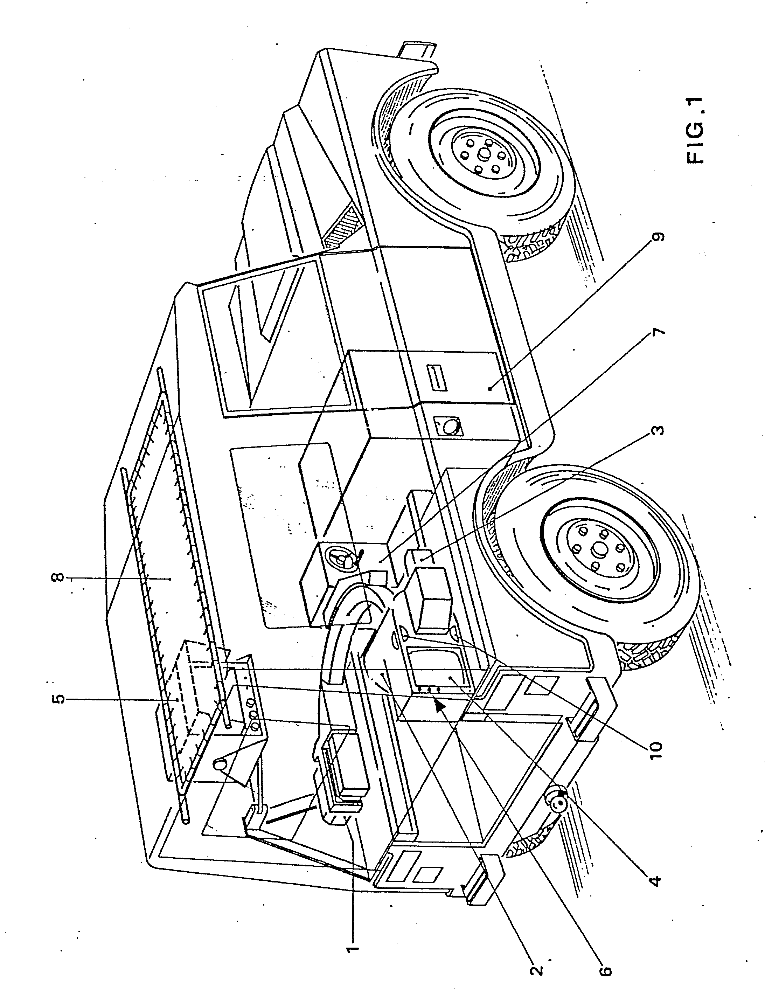

Figure 1 is a perspective view of the off-the-road vehicle showing the components of the radiological unit in their rest position;

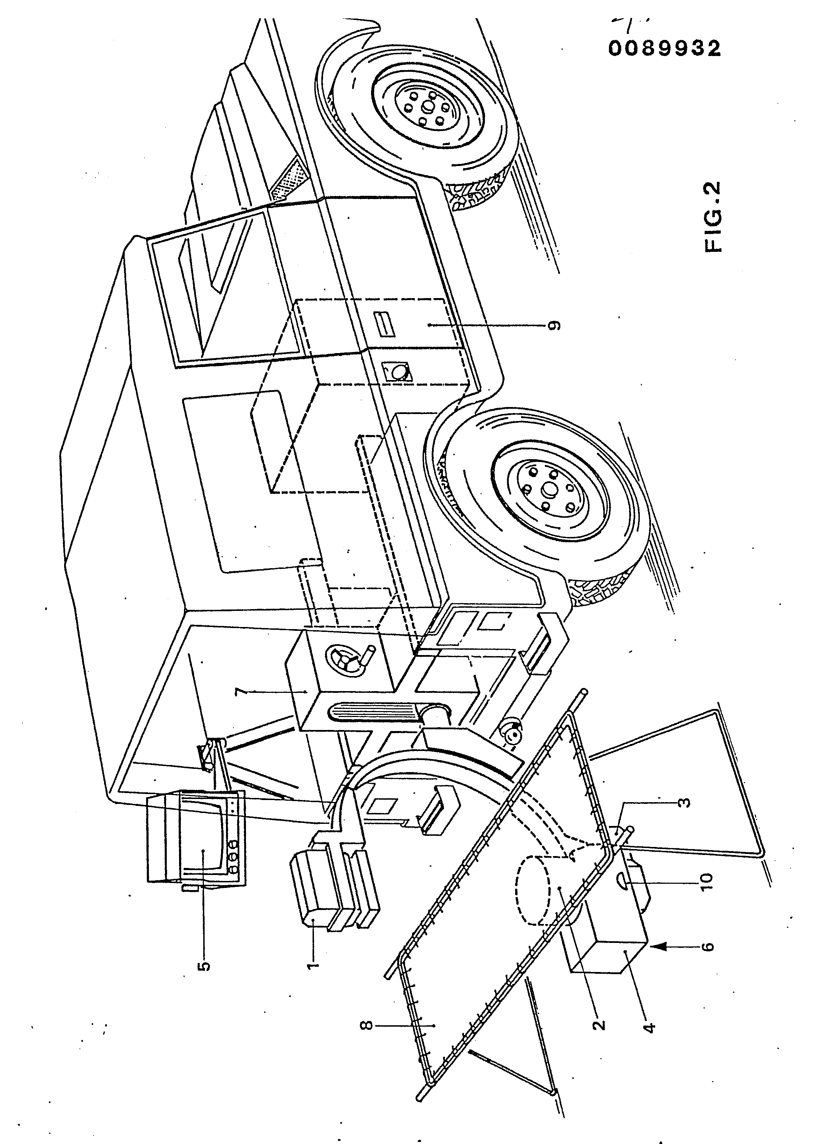

Figure 2 shows the aforesaid perspective view, but in which the components of the radiological unit are positioned for carrying out radiological examinations from below;

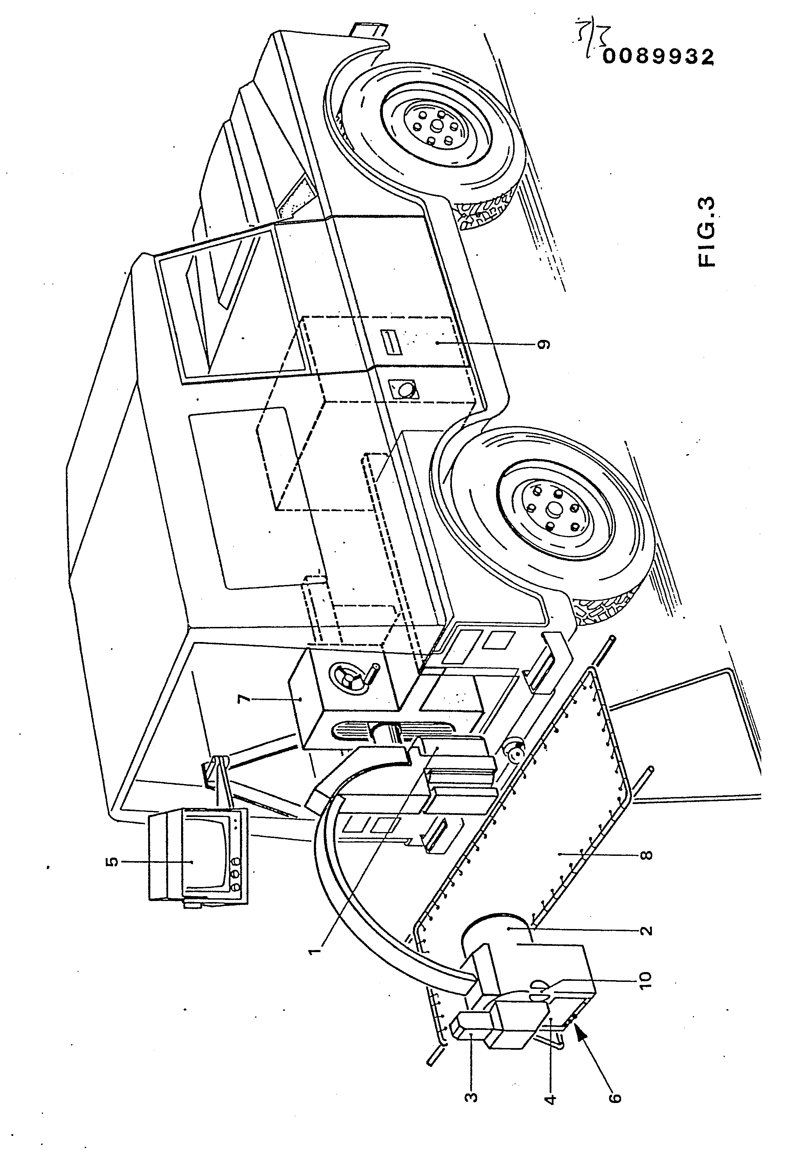

Figure 3 shows the aforesaid perspective view, but with said components positioned for carrying out lateral radiological examinations.

In the figures, the reference numeral 1 indicates the X-ray generating source, 2 the image intensifier, 3 the camera, 4 the image vision monitor, 5 the supplementary TV monitor, 6 the X-ray unit controls, 7 the mobile support with rapid manual control, 8 the bed for transporting and positioning the patient, 9 the electrical generator, and 10 the aiming handles.

It is however apparent that various modifications can be made by the expert of the art without leaving the scope of the present invention.  It is for example possible to fix the radiological unit to a framework which itself is demountably fixed to the off-the-road automobile.  It would thus be possible, using a separate generator, to enable the medical complex to operate while leaving the off-the-road automobile free to manoeuvre, for example for rescuing the wounded.

WHAT WE CLAIM IS

1. A radiological complex for selecting wounded, especially radiographically, and for providing for their treatment or despatch to the most appropriate nearby hospitals, comprising a radiological unit and an off-the-road automobile fixed to each other.

2. A complex as claimed in claim 1, wherein the medical radiological unit comprises an X-ray generating source, an image intensifier, a camera, an image vision monitor, a supplementary TV monitor, controls for the X-ray unit, a mobile support with rapid manual control, a bed for transporting and positioning the patient, aiming handles, and an electrical generator.

3. An assembly as claimed in claim 1 or 2, wherein the automobile is a Fiat 4 x 4 - 1107 AD (AR 76/A).

4. A complex as claimed in the preceding claims, wherein the radiological unit is demountably fixed to a framework which can itself be demountably fixed to the automobile, the radiological unit and the automobile being able to operate independently of each other.

5. A complex as described and illustrated in the examples.

FIG .1

1/3

0089932

5

1

8

2

4

6   10   3

7

9

0089932

FIG.2

FIG.3

0089932

# EUROPEAN SEARCH REPORT

**European Patent Office**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | FR-A- 861 821 (AERAZUR) <br> * Page 1, lines 34-53; page 2, lines 6-31, 77-84 * | 1 | A 61 B    6/00 <br> B 60 P    3/00 |
| A | GB-A- 696 538 (PICKER X-RAY CORP.) <br> * Page 1, lines 17-58 * | 1 | |
| A | FR-A-1 156 086 (J.P.D.A. ALIFA) <br> * Whole document * | 1 | |
| A | DE-C-1 213 565 (SIEMENS-REINIGER-WERKE) <br> * Column 1, line 27 - column 2, line 17; column 4, lines 14-34; figures 1-6 * | 1,4 | |
| A | DE-A-1 480 008 (SIEMENS A.G.) <br> * Page 1, line 27 - page 2, line 5; page 3, lines 8-24; page 6, claim 1; figure 1 * | 1,4 | **TECHNICAL FIELDS SEARCHED (Int. Cl. ³)** <br><br> A 61 B    6/00 <br> A 61 G    3/00 <br> B 60 P    3/00 <br> B 60 R    11/02 |
| A | US-A-3 549 885 (S.-E.L. ANDERSON/SAAB) <br> * Abstract; column 2, lines 38-61; figures 1,2 * | 2 | |

--- -/-

The present search report has been drawn up for all claims

| Place of search | Date of completion of the search | Examiner |
|---|---|---|
| THE HAGUE | 22-06-1983 | RIEB D.K. |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons
 
& : member of the same patent family, corresponding
  document

EPO Form 1503. 03.82

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl. 3) |
|---|---|---|---|
| A | US-A-3 150 260 (I.M. SMITH/MEDICAL COACHES INC.) <br> * Column 1, lines 13-32; column 4, line 63 - column 5, line 4; column 5, lines 23-29; column 6, lines 14-35 * | 1,4 | |
| | --- | | |
| A | DE-U-1 913 005 (SIEMENS-REINIGER-WERKE) <br> * Whole document * | 4 | |
| | ----- | | |

TECHNICAL FIELDS
SEARCHED (Int. Cl. 3)

The present search report has been drawn up for all claims

| Place of search <br> THE HAGUE | Date of completion of the search <br> 22-06-1983 | Examiner <br> RIEB D.K. |
|---|---|---|